# EUROPEAN PATENT APPLICATION

(11) **EP 4 782 043 A1**
(43) Date of publication of application: **29.07.2026**
(21) Application number: 24867990.4
(22) Date of filing: 19.08.2024
(51) Int. Cl.: A61M 25/00

(54) **MEDICAL DEVICE**

(30) Priority: 22.09.2023 JP 2023158123
(71) Applicant: Asahi Intecc Co., Ltd., Seto-shi, Aichi 489-0071 (JP)
(72) Inventor: TAKAHASHI, Hiroaki, Seto-shi, Aichi 489-0071 (JP); FUSEYA, Yukihiro, Seto-shi, Aichi 489-0071 (JP)
(74) Representative: Winter, Brandl - Partnerschaft mbB
(86) International application number: PCT/JP2024/029357
(87) International publication number: WO 2025/062909

(57) **Abstract**

To provide a medical device capable of sending and suctioning a liquid while forming a hollow shaft with a coil. A medical device 1 includes a hollow shaft 11 that is formed of a coil 11c in which a wire w1 is wound around a long axis and has an inner space p on a radially inward side of the coil 11c along a long axis direction, a spirally-arranged protruding portion 21 provided on an outer peripheral surface of the hollow shaft 11, and a lumen forming body 31 that is attached to the hollow shaft 11 and is provided to form a liquid-tight lumen L1.

## Description

### TECHNICAL FIELD

The present disclosure relates to a medical device.

### BACKGROUND ART

As the medical device, for example, a dilator used to dilate a hole formed in a body, a catheter used to treat a lesion in a body cavity, and the like are known.

In such a medical device, a technique of forming a hollow shaft with a coil is known (for example, see Patent Literature 1).

### CITATION LIST

### Patent Literature

Patent Literature 1: Japanese Unexamined Patent Publication No. 2007-98120

### SUMMARY OF INVENTION

### TECHNICAL PROBLEM

When performing a procedure using the above-described medical device, a physician sends a liquid such as a contrast medium or a drug solution into a body cavity, or suctions an excessive body fluid such as bile accumulated in a body. At this time, in the hollow shaft formed of the coil, since the liquid can enter and exit through a space of the coil, it is necessary for the physician to temporarily pull out the medical device formed of the coil to the outside of the body, and then insert a liquid-tight catheter to a site to be treated to perform liquid sending or suction.

The present disclosure has been made in view of the above-described circumstances and provides a medical device capable of sending and suctioning a liquid while forming a hollow shaft with a coil.

### SOLUTION TO PROBLEM

(1) A medical device including: a hollow shaft that is formed of a coil in which a wire is wound around a long axis and has an inner space on a radially inward side of the coil along a long axis direction; a spirally-arranged protruding portion provided on an outer peripheral surface of the hollow shaft; and a lumen forming body that is attached to the hollow shaft and is provided to form a liquid-tight lumen.
(2) The medical device according to (1), wherein the lumen forming body is a tube that is provided in the inner space and extends along the long axis direction.
(3) The medical device according to (2), wherein a distal end portion of the hollow shaft is rotatable around the long axis with respect to the tube.
(4) The medical device according to (2) or (3), wherein the tube and the hollow shaft are coupled so as not to be detachable.
(5) The medical device according to (2) or (3), wherein the tube and the hollow shaft are coupled so as to be detachable.
(6) The medical device according to (1), wherein the lumen forming body is a coating applied to the hollow shaft.

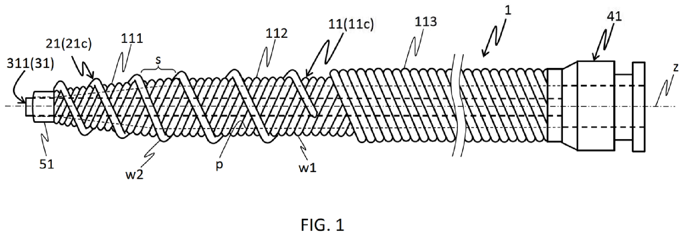

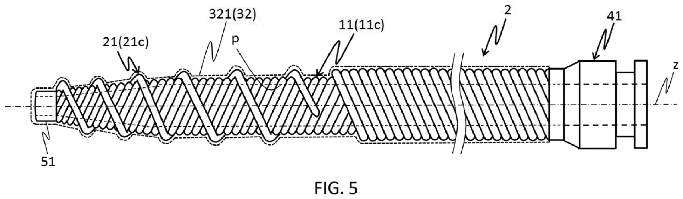

As used herein, the "long axis" refers to a center axis along the long axis direction of a hollow shaft (e.g., an axis z in FIGS. 1 and 5), unless otherwise specified. The "distal end side" refers to the direction along the long axis (long axis direction) and the direction (distal side) in which the medical device is advanced toward a treatment site. The "proximal end side" refers to the direction along the long axis (long axis direction) and the direction (proximal side) opposite to the above-described distal end side. The "distal end" refers to an end portion on the distal end side of an arbitrary member or portion, and the "proximal end" refers to an end portion on the proximal end side of an arbitrary member or portion. The "radial direction" refers to a direction orthogonal to the long axis.

### BRIEF DESCRIPTION OF DRAWINGS

FIG. 1 is a schematic side view illustrating a first embodiment.
FIG. 2A is a schematic side view of an exploded medical device in FIG. 1, illustrating an integrated structure including a distal tip, a hollow shaft, and a spirally-arranged protruding portion.
FIG. 2B is a schematic side view of the exploded medical device in FIG. 1, illustrating an integrated structure including a tube and a grip portion.
FIG. 3 is a schematic sectional view illustrating an enlarged distal end portion in FIG. 1.
FIG. 4A is a schematic sectional view illustrating an enlarged distal end portion according to a modification of the first embodiment.
FIG. 4B is a schematic sectional view illustrating an enlarged distal end portion according to a modification of the first embodiment.
FIG. 4C is a schematic sectional view illustrating an enlarged distal end portion according to a modification of the first embodiment.
FIG. 5 is a schematic side view illustrating a second embodiment.
FIG. 6 is a schematic sectional view illustrating an enlarged distal end portion in FIG. 5.
FIG. 7A is a schematic sectional view illustrating an enlarged distal end portion according to a modification of the second embodiment.
FIG. 7B is a schematic sectional view illustrating an enlarged distal end portion according to a modification of the second embodiment.
FIG. 7C is a schematic sectional view illustrating an enlarged distal end portion according to a modification of the second embodiment.

### DESCRIPTION OF EMBODIMENTS

Hereinafter, embodiments of the present disclosure will be described with reference to the drawings. The present disclosure is not limited to the embodiments described in the drawings. The dimensions of each part illustrated in the drawings are the dimensions illustrated to facilitate understanding of the embodiments, and do not necessarily correspond to the actual dimensions.

In each drawing, the left side in the drawing is a distal end side (distal side) that is inserted into a deeper portion in a body, and the right side is a proximal end side (proximal side, hand side) that is operated by a professional such as a physician.

### <First Embodiment>

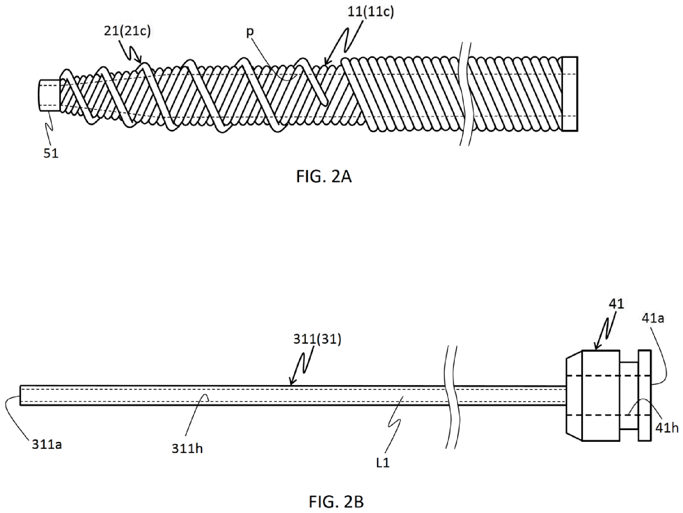

FIGS. 1 to 3 are schematic views illustrating a first embodiment. The medical device 1 is a dilator. As illustrated in FIGS. 1, 2A, and 2B, the medical device 1 generally includes a hollow shaft 11, a spirally-arranged protruding portion 21, a lumen forming body 31, a grip member 41, and a distal tip 51.

The hollow shaft 11 is a member that is formed of a coil in which a wire is wound around the long axis and has an inner space on the radially inward side of the coil along the long axis direction.

The hollow shaft includes a tapered portion 111, a large diameter portion 112, and a main body portion 113. The tapered portion 111 is a tapered portion having a shape tapered toward the distal end side. The large diameter portion 112 is a portion that has a distal end located at the proximal end of the tapered portion 111, extends toward the proximal end side in the long axis direction, and has a substantially constant outer diameter. The main body portion 113 is a portion that has a distal end located at the proximal end of the large diameter portion 112 and that extends toward the proximal end side in the long axis direction.

Each of the tapered portion 111, the large diameter portion 112, and the main body portion 113 is formed by appropriately adjusting the shape of the outermost periphery of the coil (hereinafter, also referred to as a "first coil 11c") (the diameter of the first coil 11c) forming the hollow shaft 11 along the long axis direction. The first coil 11c is formed by spirally winding a wire w1 so that the wires w1 adjacent to each other along the long axis direction of the hollow shaft 11 are in close contact with each other.

The first coil 11c may be either a single-thread coil formed by winding a single wire or a multi-thread coil formed by winding two or more wires. Examples of the wire include a single wire (one single wire) and a twisted wire (a bundle of wire groups formed by twisting two or more single wires together in advance).

Since the first coil 11c is inserted into a body cavity, the first coil 11c is preferably antithrombogenic, flexible, and biocompatible. Examples of the material of the wire w1 forming the first coil 11c include resin materials such as polyamide resins, polyolefin resins, polyester resins, polyurethane resins, silicone resins, and fluororesins, and metallic materials such as stainless steels (e.g., SUS304) and superelastic alloys (e.g., nickel-titanium alloys).

As described above, since the hollow shaft 11 is formed of the first coil w1 in which the wire 11c is wound around the long axis, it is possible to improve the flexibility of the hollow shaft 11 and the torquability of the first coil 11c. The torquability is the ability to transmit a rotational force applied to the proximal end portion (the grip member 41 described below according to the present embodiment) of the hollow shaft 11 to the distal end portion of the hollow shaft 11.

The spirally-arranged protruding portion 21 is a member that is provided on the outer peripheral surface of the hollow shaft and has a space between adjacent portions along the long axis direction of the hollow shaft. The spirally-arranged protruding portion 21 can be formed of a coil (hereinafter, also referred to as a "second coil 21c"). For example, the second coil 21c may be provided to protrude toward the outer side in the radial direction of the hollow shaft 11 from the outer peripheral surface of the hollow shaft 11 by winding a wire w2 around the outer peripheral surface of the hollow shaft 11 (the outer peripheral surfaces of the tapered portion 111 and the large diameter portion 112 according to the present embodiment) and to have a space s by separating the adjacent wires w2 from each other in the long axis direction.

Since the second coil 21c is inserted into a body cavity, the second coil 21c is preferably antithrombogenic, flexible, and biocompatible. Examples of the material of the wire w2 forming the second coil 21c include the same materials as those exemplified as the material of the wire w1 forming the first coil 11c.

The first coil 11c and the second coil 21c can be joined to each other by, for example, brazing, welding, fixing with an adhesive, welding with coating, or the like, at the end portions thereof.

As described above, since the spirally-arranged protruding portion 21 is provided on the outer peripheral surface of the hollow shaft 11 and has the space s between the adjacent portions along the long axis direction of the hollow shaft 11, the medical device 1 can be advanced by the rotation operation due to the screw action of the spirally-arranged protruding portion 21. The spirally-arranged protruding portion 21 does not hinder the forward movement by a pushing operation as long as it is not engaged with the inner peripheral surface of the body cavity.

The lumen forming body 31 is attached to the hollow shaft and forms a liquid-tight lumen on the radially inward side of the hollow shaft. The lumen forming body 31 according to the present embodiment is a liquid-tight tube 311 that is provided in an inner space p of the first coil 11c and extends along the long axis direction. The tube 311 includes an inner cavity 311h having an opening portion 311a at the distal end. The inner cavity 311h is kept to be liquid-tight so that the liquid does not leak to the outside of the tube 311 or does not flow in from the outside. Thus, the medical device 1 has high liquid tightness with a simple configuration.

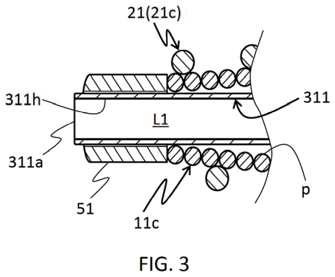

The outer peripheral surface of the tube 311 may be in contact with or may be separated from the inner peripheral surface of the first coil 11c. As illustrated in FIG. 3, according to the present embodiment, the outer peripheral surface of the tube 311 abuts on the inner peripheral surface of the distal end portion of the first coil 11c, and the distal end portion of the tube 311 is provided to protrude from the distal end of the distal tip 51 through the distal tip 51 described below.

The tube 311 and the hollow shaft 11 may be coupled so as not to be detachable. The tube 311 and the hollow shaft 11 may be coupled to each other via the grip member 41 by, for example, fixing each of the tube 311 and the first coil 11c to the grip member 41. Thus, the tube 311 and the hollow shaft 11 can always be used integrally.

The tube 311 and the hollow shaft 11 may be coupled so as to be detachable. The mechanism for attachment and detachment is not particularly limited. For example, the grip member to which the tube is fixed may be coupled to the first coil 11c by using an attaching and detaching mechanism (not illustrated). As a result, the tube 311 can be inserted into the hollow shaft 11 or both can be separated from each other as needed, and liquid sending of a contrast medium or the like and suction of a body fluid or the like can be performed as appropriate.

Since the tube 311 is inserted into a body cavity, the material forming the tube 311 is preferably antithrombogenic, flexible, and biocompatible. The material forming the tube 311 is preferably flexible to be able to follow a curved body cavity. Examples of the material forming the tube 311 include polyamide resins, polyolefin resins, polyester resins, polyurethane resins, silicone resins, fluororesins, polyacrylic acids, polyimide resins, and polyamide-imide resins.

The grip member 41 is a member used for the medical device 1 to be pushed into the body or rotated by the professional. The grip member 41 according to the present embodiment has an inner cavity 41h having an opening portion 41a at the proximal end and is fixed to the proximal end portion of the tube 311.

The inner cavity 311h of the tube 311 and the inner cavity 41h of the grip member 41 communicate with each other, and a lumen L1 is formed by the inner cavity 311h and the inner cavity 41h. For example, a guide wire is inserted into the lumen L1 during a procedure, or a contrast medium to be injected into a body cavity or a liquid such as a body fluid suctioned from a body cavity flows through the lumen L1.

The proximal end of the distal tip 51 is fixed to the distal end of the hollow shaft 11. The distal tip 51 is a substantially hollow cylindrical member extending from the distal end of the hollow shaft 11 toward the distal end side. The distal tip 51 may have, for example, a flatter surface than the outer peripheral surface of the first coil 11c (the uneven surface formed by the wire w1) and may be formed to have a hollow shape so that the tube 311 can be inserted. The distal tip 51 can be formed, for example, at the distal end portion of the first coil 11c by forming its surface into a flat shape using a brazing material such as silver-tin brazing or gold-tin brazing. As a result, the insertability of the hollow shaft 11 into a hole drilled in advance can be improved, and the procedure can proceed smoothly.

The distal end portion of the hollow shaft 11 may be provided to be rotatable around the long axis with respect to the tube 311. The inner peripheral surface of the distal end portion of the first coil 11c and the outer peripheral surface of the tube 311 do not need to be fixed (joined) to each other, and the first coil 11c may be provided to be freely rotatable around the tube 311. As a result, high torquability can be maintained up to the distal end portion of the medical device 1. According to the present embodiment, the inner peripheral surface of the distal tip 51 is not fixed to the outer peripheral surface of the tube 311. As a result, the first coil 11c and the distal tip 51 can freely rotate integrally around the tube 311.

An example of the use mode of the medical device 1 exemplified as a medical device will be described.

First, the physician uses an introducer needle (not illustrated) to penetrate and puncture the target body tissue. Subsequently, the physician inserts a guide wire (not illustrated) into the inner cavity of the introducer needle and then removes the introducer needle from the body.

Subsequently, the physician inserts the proximal end of the guide wire into the inner cavity 311h from the opening portion 311a of the tube 311 and then advances the medical device 1 in the body cavity along the guide wire to insert the distal tip 51 into the hole. Subsequently, the physician expands the hole by advancing the medical device 1 while rotating the hollow shaft 11 by operating the grip member 41. At this time, since the tapered portion 111 advances to the distal end side while passing through the hole by the screw action or the like of the spirally-arranged protruding portion 21 (the second coil 21c) due to the rotation of the hollow shaft 11, the hole is expanded by the tapered portion 111.

Subsequently, the physician sends a liquid such as a contrast medium or suctions a liquid such as a body fluid through the lumen L1. To be specific, the physician couples a syringe (e.g., a contrast medium injection device or a liquid suction device) to the opening portion 41a of the grip member 41 using a coupling member such as a Luer lock (not illustrated), and sends or suctions a liquid through the lumen L1. At this time, since the tube 311 is provided such that the inner cavity 311h forms the liquid-tight lumen L1, the liquid in the lumen L1 does not leak to the outside, and liquid sending and suction are performed.

After the liquid sending and the suction are completed, the physician retracts the medical device 1 while reversely rotating the hollow shaft 11 by the operation of the grip member 41 and removes the medical device 1 to the outside of the body.

As described above, since the medical device 1 has the above-described configuration, the medical device 1 can exhibit excellent torquability and has a function of sending a liquid such as a contrast medium or suctioning a liquid such as a body fluid through the lumen L1 formed in a liquid-tight manner by the tube 311.

Unlike the above-described embodiment, the tube 311 and the hollow shaft 11 may be detachably coupled to each other using, for example, a known attaching and detaching mechanism so that the tube and the hollow shaft can be attached to and detached from each other.

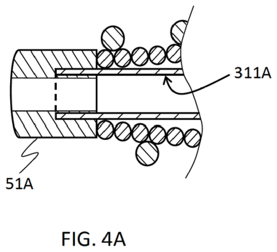

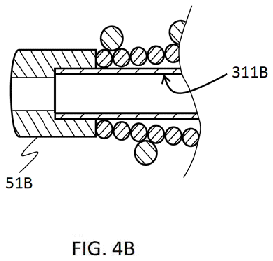

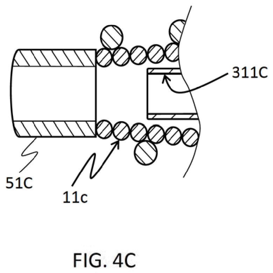

Unlike the above-described embodiment, the distal end portion of the tube may be provided so as not to protrude from the distal tip. For example, as illustrated in FIGS. 4A and 4B, distal end portions of tubes 311A and 311B may be provided to simply abut on distal tips 51A and 51B without being fixed thereto. As illustrated in FIG. 4C, the distal end portion of a tube 311C may be provided away from a proximal end of a distal tip 51C toward the proximal end side. That is, the distal end of the tube 311C may be located at an intermediate position of the first coil 11c in the long axis direction.

### <Second Embodiment>

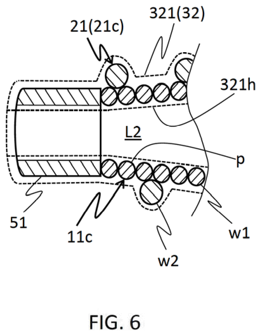

FIGS. 5 and 6 are schematic views illustrating a second embodiment. The medical device 2 is a dilator. As illustrated in FIG. 5, the medical device 2 schematically includes the hollow shaft 11, the spirally-arranged protruding portion 21, a lumen forming body 32, the grip member 41, and the distal tip 51. The medical device 2 according to the present embodiment is different from the first embodiment in the configuration of the lumen forming body 32. Since the configurations of the hollow shaft 11, the spirally-arranged protruding portion 21, the grip member 41, and the distal tip 51 are the same as those in the first embodiment, the same portions are denoted by the same symbols, and the detailed description thereof will be omitted. Since the usage mode of the medical device 2 is the same as that of the first embodiment, a detailed description thereof will also be omitted.

The lumen forming body 32 is attached to the hollow shaft 11 and is provided to form a liquid-tight lumen. The lumen forming body 32 according to the present embodiment is a coating 321 applied to the hollow shaft 11.

The coating 321 is formed to cover at least a part of the hollow shaft 11 so that a liquid does not flow in or out at least through the interval between the adjacent wires w1 of the first coil 11c. According to the present embodiment, an inner cavity 321h formed on the radially inward side of the coating 321 forms a liquid-tight lumen L2. For example, as illustrated in FIG. 6, the portion where the coating 321 is provided is a portion that covers the entirety of the first coil 11c, the spirally-arranged protruding portion 21, and the distal tip 51.

From the viewpoint of ensuring the torquability, the material of the coating 321, the thickness of the coating 321, and the like are preferably determined such that the relative movement between the adjacent wires w1 of the first coil 11c is not hindered. Since the coating 321 is inserted into a body cavity, the material is preferably antithrombogenic, flexible, and biocompatible.

Examples of the material forming the coating 321 include resins such as polyamide resins, polyolefin resins, polyester resins, polyurethane resins, silicone resins, fluororesins, polyacrylic acid, polyimide resins, and polyamide-imide resins.

As described above, since the medical device 2 has the above-described configuration, the medical device 2 can be operated while exhibiting excellent torquability, and provides a function of sending a liquid such as a contrast medium or suctioning a liquid such as a body fluid through the lumen L2 formed in a liquid-tight manner with the coating 321.

In the medical device 2, since the lumen forming body 32 is formed of the coating 321, the wide inner cavity 321h can be secured in the inner space p by reducing the thickness of the coating 321 while maintaining the liquid tightness. As a result, the medical device 2 has a function of smoothly sending and suctioning the liquid.

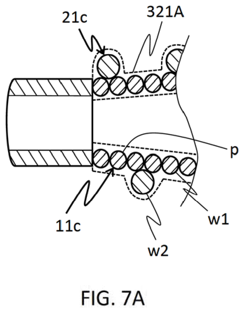

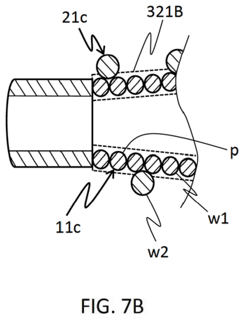

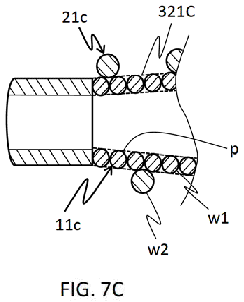

The portion where the coating is provided is not limited to the portion according to the above-described embodiment. Examples of the portion where the coating is provided include a portion that covers only the entirety of the first coil 11c and the second coil 21c (see FIG. 7A), a portion that covers only the entirety of the first coil 11c (see FIG. 7B), and a portion that covers the interval between the adjacent wires w1 of the first coil 11c (see FIG. 7C).

The present disclosure is not limited to the configurations according to the above-described embodiments, but is defined by the scope of the claims, and is intended to include all modifications within the meaning and scope equivalent to the scope of the claims. A part of the configuration according to the above-described embodiment may be deleted or replaced with another configuration, and another configuration may be added to the configuration according to the above-described embodiment.

For example, unlike the embodiments described above, the medical device may be a device (e.g., catheter) which have a substantially constant outer diameter along the long axis.

Unlike the embodiment described above, the medical device may be a device which includes both a tube and a coating. Examples of such a medical device include a medical device in which the distal end portion of the tube is provided to be separated from the proximal end of the distal tip toward the proximal end side and at least a portion of the first coil located between the proximal end of the distal tip and the distal end of the tube is covered with a coating. That is, the distal end of the tube may be located at an intermediate position of the first coil in the long axis direction.

### DESCRIPTION OF REFERENCE NUMERALS

1, 2 Medical device (dilator)
11 Hollow shaft
11c First coil
21 Spirally-arranged protruding portion
21c Second coil
31, 32 Lumen forming body
311, 311A, 311B, 311C Tube
311h Inner cavity
321 Coating
321h Inner cavity
41 Grip member
51 Distal tip
w1 Wire
w2 Wire
s Space
p Inner space
L1, L2 Lumen

## Claims

1. A medical device comprising:
a hollow shaft that is formed of a coil in which a wire is wound around a long axis and has an inner space on a radially inward side of the coil along a long axis direction;
a spirally-arranged protruding portion provided on an outer peripheral surface of the hollow shaft; and
a lumen forming body that is attached to the hollow shaft and is provided to form a liquid-tight lumen.

2. The medical device according to claim 1, wherein the lumen forming body is a tube that is provided in the inner space and extends along the long axis direction.

3. The medical device according to claim 2, wherein a distal end portion of the hollow shaft is rotatable around the long axis with respect to the tube.

4. The medical device according to claim 2 or 3, wherein the tube and the hollow shaft are coupled so as not to be detachable.

5. The medical device according to claim 2 or 3, wherein the tube and the hollow shaft are coupled so as to be detachable.

6. The medical device according to claim 1, wherein the lumen forming body is a coating applied to the hollow shaft.
